# EUROPEAN PATENT SPECIFICATION

(11) **EP 2 408 299 B1**
(45) Date of publication and mention of the grant of the patent: **23.10.2013**
(21) Application number: 10754148.4
(22) Date of filing: 19.03.2010
(51) Int. Cl.: A01N 31/08, A61K 31/05

(54) **FATTY ACID MONOGLYCERIDE COMPOSITIONS**
FETTSÄURE-MONOGLYCERID-ZUSAMMENSETZUNGEN
COMPOSITIONS DE MONOGLYCÉRIDES D'ACIDES GRAS

(30) Priority: 28.05.2009 US 213313 P; 20.03.2009 US 202635 P
(43) Date of publication of application: 25.01.2012
(73) Proprietor: Stiefel Laboratories, Inc., Coral Gables, FL 22134 (US); Stiefel Research Australia Pty Ltd, Rowville VIC 3178 (AU)
(72) Inventor: LENN, Jon, Palo Alto CA 94304 (US); HUNT, Barry, Rowville VIC 3178 (AU); HSIA, Edward, Palo Alto CA 94304 (US); HOFLAND, Hans, Palo Alto CA 94304 (US)
(74) Representative: Reeves, Julie Frances
(86) International application number: PCT/US2010/027905
(87) International publication number: WO 2010/108060

(56) References cited:
- EP-A2- 0 465 423
- WO-A2-2006/017594
- DE-A1- 4 434 781
- DE-A1- 19 540 465
- US-A- 4 067 997
- US-A1- 2005 165 104
- RUZICKA JAN ET AL: "Antimicrobial effects of 1-monoacylglycerols prepared by catalytic reaction of glycidol with fatty acids.", EUROPEAN FOOD RESEARCH AND TECHNOLOGY, vol. 217, no. 4, October 2003 (2003-10), pages 329-331, XP002679225, ISSN: 1438-2377
- PLOCKOVA M ET AL: "INHIBITION OF FOOD MOULDS BY LAURIC ACID DERIVATIVES", ADVANCED IN FOOD SCIENCES, TECHNISCHE UNIVERSITAET MUENCHEN, MUENCHEN, DE, vol. 21, no. 1/02, 1 January 1999 (1999-01-01), pages 44-47, XP009007349, ISSN: 1431-7737

## Description

### FIELD OF THE INVENTION

The present invention relates to topical pharmaceutical compositions for use in treating or preventing an infection caused by fungi.

### BACKGROUND OF THE INVENTION

Fungal infections of the nail (known as onychomycosis) and surrounding skin are unsightly and, in certain cases, can result in loss of the nail. These infections are caused by dermatophytes, Candida and non-dermatophytic moulds. Common dermatophytes which infect the nail and surrounding skin include *Trichophyton rubrum* and *Trichophyton mentagrophytes.*

Current treatment options for onychomycosis include oral treatment with terbinafine, itraconazole, griseofulvin or fluconazole. Alternative options include topical treatment with amorolfine or ciclopirox. One such topical composition is described in US 4,957,730 which discloses a nail varnish comprising a water insoluble film forming component and a 1-hydroxy-2-pyridone derivative (such as ciclopirox).

US 6,455,592 to Laugier et al. teaches a composition comprising a pharmacologically effective amount of terbinafine hydrochloride, a solvent medium comprising water and at least one straight- or branched-chain C₂-C₈ alkanol, and a hydrophilic penetration agent.

US Published Patent Application No. 2003/0190340 to Bohn et al. discloses a preparation comprising a hydrophilic gel-forming agent, water and a compound such as 1-hydroxy-4-methyl-6-cyclohexyl-2(1H)pyridone.

US Published Patent Application No. 2004/0028721 to Colombo et al. discloses single-layer films for the dermal or transdermal administration of active ingredients comprising an active ingredient, a film-forming agent and a hydrophilic adhesive polymer.

US 6,596,763 to Thormar et al*.* teaches use of lipids, including moncaprin for treatment of HSV-1 infections.

German DE 195 40 465 A1 to Wolf et al. discloses mono-, di- and tri-glycerol monocarboxylic acid esters as antimycotic agents for the treatment of yeast infections, specifically *Pityrosporum ovale.*

There remains a need in the art for topical compositions that are effective in treating or preventing onychomycosis and other fungal conditions. The present invention addresses these needs.

### SUMMARY OF THE INVENTION

According to one aspect, the present invention provides a pharmaceutical composition for use in the topical treatment or prophylaxis of a fungal condition caused by *Trichophyton rubrum,* comprising a therapeutically effective amount of a fatty acid monoester of glycerol of the general formula Ia 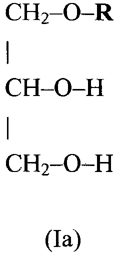 wherein **R** is a branched chain or straight chain acyl group having from 4 to 22 carbon atoms (i.e. -C(O)-C₄₋₂₂), and a pharmaceutically acceptable carrier or diluent thereof, and optionally a second pharmaceutically active agent.

According to another aspect, the present invention provides a pharmaceutical composition for use in a method for the treatment or prophylaxis of a fungal condition caused by *Trichophyton rubrum* in a patient, the method comprising topically applying to the patient a pharmaceutically acceptable composition comprising a therapeutically effective amount of a fatty acid monoester of glycerol of the general formula Ia 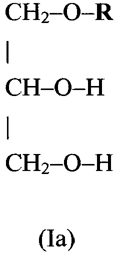 wherein **R** is a branched chain or straight chain acyl group having from 4 to 22 carbon atoms (i.e. -C(O)-C₄₋₂₂), and a pharmaceutically acceptable carrier or diluent thereof, and optionally a second pharmaceutically active agent.

According to a further aspect, the present invention relates to the use of the compositions described herein for the preparation of a medicament for the treatment or prophylaxis of a fungal condition caused by *Trichophyton rubrum.*

### BRIEF DESCRIPTION OF THE DRAWINGS

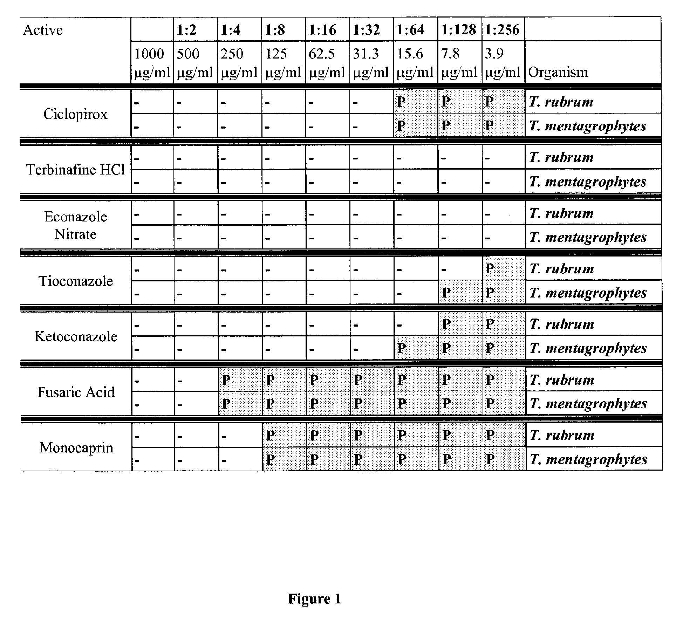

Figure 1 depicts the minimal inhibitory concentrations (MIC) of various antifungal compounds in liquid (broth) cultures of *Trichophyton rubrum* and *Trichophyton mentagrophytes* as described in Example 1. "P" indicates that the culture was found positive for fungal growth, i.e. more than 2 colonies were found per plate; "-" indicates that no growth was observed.

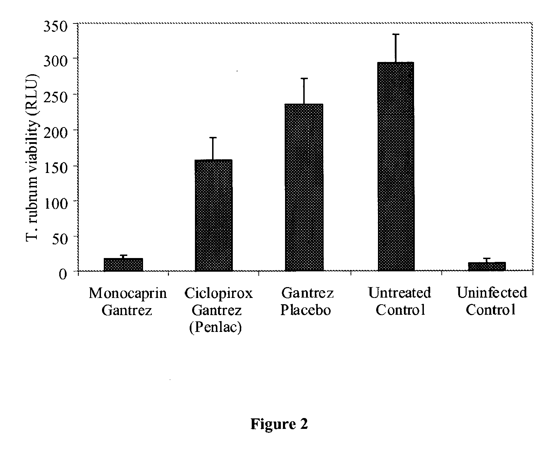

Figure 2 identifies viability of *Trichophyton rubrum* in the Infected Nail Model (described in Example 2) after application of 1-monocaprin nail lacquer prepared in accordance with Example 3 and PENLAC® nail lacquer, along with placebo and control samples. The bars represent mean ± SEM, n=6.

### DETAILED DESCRIPTION OF THE INVENTION

According to one aspect, the present invention provides a pharmaceutical composition for use in the topical treatment or prophylaxis of a fungal condition caused by *Trichophyton rubrum,* comprising a therapeutically effective amount of a fatty acid monoester of glycerol of the general formula Ia 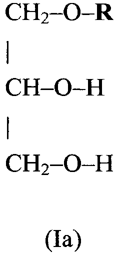 wherein **R** is a branched chain or straight chain acyl group having from 4 to 22 carbon atoms (i.e. -C(O)-C₄₋₂₂), and a pharmaceutically acceptable carrier or diluent thereof, and optionally a second pharmaceutically active agent.

According to another aspect, the present invention provides a pharmaceutical composition for use in a method for the treatment or prophylaxis of a fungal condition caused by *Trichophyton rubrum,* the method comprising topically applying to the patient a pharmaceutically acceptable composition comprising a therapeutically effective amount of a fatty acid monoester of glycerol of the general formula Ia 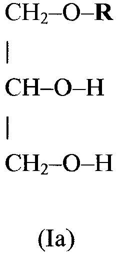 wherein **R** is a branched chain or straight chain acyl group having from 4 to 22 carbon atoms (i.e. -C(O)-C₄₋₂₂), and a pharmaceutically acceptable carrier or diluent thereof, and optionally a second pharmaceutically active agent.

According to a further aspect, the present invention relates to the use of the compositions described herein for the preparation of a medicament for the treatment or prophylaxis of a fungal condition caused by *Trichophyton rubrum.*

According to one embodiment, R is a branched chain or straight chain acyl group having from 8 to 14 carbon atoms (i.e. -C(O)-C₈₋₁₄).

In another embodiment, **R** is a straight chain acyl group having from 8 to 14 carbon atoms, selected from the group consisting of:
-C(O)-C₇H₁₅ (C₈ from octanoic acid),
-C(O)-C₈H₁₇ (C₉ from nonanoic acid),
-C(O)-C₉H₁₉ (C₁₀ from decanoic acid),
-C(O)-C₁₀H₂₁ (C₁₁ from undecanoic acid),
-C(O)-C₁₁H₂₃ (C₁₂ from dodecanoic acid),
-C(O)-C₁₂H₂₅ (C₁₃ from tridecanoic acid), and
-C(O)-C₁₃H₂₇ (C₁₄ from tetradecanoic acid).

In another embodiment, **R** is a straight chain acyl group selected from the group consisting of -C(O)-C₉H₁₉(C₁₀), -C(O)-C₁₀H₂₁(C₁₁) and -C(O)-C₁₁H₂₃(C₁₂).

In one embodiment, **R** is -C(O)-C₉H₁₉.

In another embodiment, **R** is-C(O)-C₁₀H₂₁.

In another embodiment, **R** is -C(O)-C₁₁H₂₃.

According to another embodiment, the composition comprises a fatty acid monoester of glycerol of the general formula (Ia).

According to yet another embodiment, the composition comprises a fatty acid monoester of glycerol of the general formula (Ia) and **R** is a straight chain acyl group having from 8 to 14 carbon atoms selected from the group consisting of:
-C(O)-C₇H₁₅ (C₈ from octanoic acid),
-C(O)-C₈H₁₇ (C₉ from nonanoic acid),
-C(O)-C₉H₁₉ (C₁₀ from decanoic acid),
-C(O)-C₁₀H₂₁ (C₁₁ from undecanoic acid),
-C(O)-C₁₁H₂₃ (C₁₂ from dodecanoic acid),
-C(O)-C₁₂H₂₅ (C₁₃ from tridecanoic acid), and
-C(O)-C₁₃H₂₇ (C₁₄ from tetradecanoic acid).

According to yet another embodiment, the composition comprises a fatty acid monoester of glycerol of the general formula (Ia) and **R** is a straight chain acyl group selected from the group consisting of -C(O)-C₉H₁₉(C₁₀), -C(O)-C₁₀H₂₁(C₁₁) and -C(O)-C₁₁H₂₃(C₁₂). That is, according to this embodiment, the fatty acid monoester of glycerol is selected from the group consisting of 1-decanoyl-rac-glycerol (C₁₀), 1-undecanoyl-rac-glycerol (C₁₁) and 1-lauroyl-rac-glycerol (C₁₂).

In one embodiment, the composition comprises a fatty acid monoester of glycerol of the general formula (Ia) and **R** is a straight chain acyl group which is -C(O)-C₉H₁₉. That is, according to this embodiment, the fatty acid monoester of glycerol is 1-decanoyl-rac-glycerol (C₁₀), also known as 1-monocaprin. In another embodiment, the fatty acid monoester is 1-undecanoyl-rac-glycerol (C₁₁). In yet another embodiment, the monoester is 1-lauroyl-rac-glycerol (C₁₂), also known as 1-monolaurin.

According to an embodiment, the fatty acid monoester of glycerol is present in an amount from about 0.1% to about 20% by weight. In one embodiment, the fatty acid monoester is present in an amount from about 1% to about 10% by weight. In another embodiment, the monoester is present in an amount of about 1%, 2%, 3%, 4%, 5%, 6%, 7%, 8%, 9% or 10% by weight.

The pharmaceutical compositions of a fatty acid monoester of glycerol of the general formula Ia can be formulated as a nail lacquer, enamel, paint, solution, lotion, cream, gel, aerosol foam, aerosol spray, or as any other suitable pharmaceutically acceptable topical dosage form. According to an embodiment, the pharmaceutical composition is a nail lacquer. According to an alternative embodiment, the pharmaceutical composition is an aerosol foam.

According to an embodiment, the pharmaceutical composition is free or substantially free of water.

### Nail lacquer

According to an embodiment, the pharmaceutical composition further comprises at least one volatile solvent and a film forming component. According to this embodiment, the composition is a nail lacquer. Topical application of the nail lacquer allows a flexible film to be deposited onto the affected area i.e. once the volatile solvent has evaporated. This flexible film protects the infected area from environmental stresses and/or clothing, and acts as a reservoir for the active ingredient.

### Volatile solvent

The nail lacquer of the present invention comprises one or more volatile solvents. Thus, when the nail lacquer is administered to the nail and surrounding skin of a patient, the evaporation of the volatile solvent leaves a matrix of the active ingredient within the film forming component on the surface of the nail and surrounding skin. This in turn permits the ready penetration of the active ingredient into the nail and skin.

Suitably, the volatile solvent is selected from ethanol, propyl alcohol, isopropyl alcohol, n-butyl alcohol, t-butyl alcohol, butoxy ethanol, acetone, ethyl acetate, butyl acetate, or a combination or mixture thereof. According to an embodiment, the volatile solvent is a mixture of ethanol and ethyl acetate. According to an alternative embodiment, the volatile solvent is a mixture of isopropyl alcohol and ethanol. According to an embodiment, the volatile solvent is present in an amount from about 40% to about 99.85% by weight. According to another ambodiment, the volatile solvent is present in an amount from about 60% to about 90% by weight. According to yet another embodiment, the volatile solvent is present in an amount from about 75% to about 85% by weight.

### Film forming component

According to an embodiment of the present invention, the film forming component is a film forming polymer. Suitable film forming polymers include hydroxypropylmethyl cellulose, hydroxypropyl cellulose, polyvinyl pyrrolidone, carbomer, PVM/MA decadiene cross polymer, hydroxypropylguar, octylacrylamide acrylates copolymer, aminoalkyl methacrylate copolymer, ammonio methacrylate copolymer, PVP/VA copolymers, PVA, a C₂ - C₄ alkyl ester of PVM/MA copolymer, shellac, or a combination or mixture thereof.

According to one embodiment, the film forming polymer is a hydrophilic polymer. Suitably, the hydrophilic polymer is selected from hydroxypropylmethyl cellulose, hydroxypropyl cellulose, polyvinyl pyrrolidone, carbomer, PVM/MA decadiene cross polymer or hydroxypropylguar.

According to an alternative embodiment, the film forming polymer is a hydrophobic polymer. Suitably, the hydrophobic polymer is selected from octylacrylamide acrylates copolymer, aminoalkyl methacrylate copolymer, ammonio methacrylate copolymer, PVP/VA copolymer, PVA, a C₂-C₄ alkyl ester of PVM/MA copolymer, or shellac.

According to an embodiment, the C₂ - C₄ alkyl ester of PVM/MA copolymer is the ethyl ester, isopropyl ester or butyl ester. According to one embodiment, the film forming component is the butyl ester of PVM/MA copolymer.

According to an embodiment, the film forming component is present in an amount from about 0.05% to about 40% by weight. In another embodiment, the film forming component is present in an amount from about 0.1% to about 25% by weight. According to yet another embodiment, the film forming component is present in an amount from about 10% to about 20% by weight.

For "once daily" treatment, it is preferred that a hydrophilic polymer is used as the film forming component. For "once weekly" treatment, it is preferred that a hydrophobic polymer is used as the film forming component. Use of such a hydrophobic polymer creates a water- and rub-resistant film suitable for extended treatment (i.e. less frequent application).

### Aerosol foam composition

According to an alternative embodiment, the pharmaceutical composition further comprises water, a surfactant component, and a propellant. According to this embodiment, the composition is an aerosol foam composition.

According to an embodiment, the water is present in the composition in an amount from about 80% to about 96% by weight. In another embodiment, water is present in an amount from about 90% to about 95% by weight, such as about 90%, 91%, 92%, 93%, 94% or 95% by weight.

The present topical aerosol foam compositions comprise a surfactant component. It is believed that the surfactant will emulsify the fatty acid monoester of glycerol in the water component i.e. to form an oil-in-water emulsion.

Suitably, the surfactant is present in the composition in an amount from about 0.01% to about 10% by weight. In another embodiment, the surfactant is present in an amount from about 0.25% to about 2% by weight, such as about 0.25%, 0.5%, 0.75%, 1%, 1.25%, 1.5%, 1.75% or 2% by weight.

A surfactant's hydrophilic/lipophilic balance (HLB) describes the surfactant's affinity toward water or oil. The HLB scale ranges from 1 (totally lipophilic) to 20 (totally hydrophilic), with 10 representing an equal balance of both characteristics. Lipophilic surfactants tend to form water-in-oil (w/o) emulsions, and hydrophilic surfactants tend to form oil-in-water (o/w) emulsions. The HLB of a blend of two surfactants equals the weight fraction of surfactant A times its HLB value plus the weight fraction of surfactant B times its HLB value (weighted average).

In one embodiment, the surfactant component comprises a hydrophilic surfactant. Suitably, the surfactant component is free or substantially free of a lipophilic surfactant.

In another embodiment, the hydrophilic surfactant is a non-ionic hydrophilic surfactant. In one embodidiment, the non-ionic hydrophilic surfactant is a hydrophilic ethoxylated fatty alcohol ether or a hydrophilic sorbitan derivative.

According to an embodiment, the non-ionic hydrophilic surfactant is a hydrophilic ethoxylated fatty alcohol ether. Suitably, the hydrophilic ethoxylated fatty alcohol ether is selected from the group consisting of steareth-10, steareth-20, ceteareth-6, ceteareth-10, ceteareth-12, ceteareth-15, ceteareth-20, ceteareth-21, ceteareth-22, ceteareth-25, ceteareth-30, ceteareth-31, ceteareth-32, ceteareth-33, laureth-5, laureth-9, laureth-10, laureth-12, laureth-15, laureth-20, laureth-21, laureth-22, laureth-23, nonoxynol-9, oleth-10, oleth-20, and mixtures thereof.

According to an embodiment, the hydrophilic ethoxylated fatty alcohol ether is ceteareth-20.

In another embodiment, the non-ionic hydrophilic surfactant is a hydrophilic sorbitan derivative. Suitably, the hydrophilic sorbitan derivative is selected from polysorbate 20, polysorbate 40, polysorbate 60 or polysorbate 80, or a combination or mixture thereof.

The present topical aerosol foam compositions comprise a propellant in order to produce the foam upon actuation. According to an embodiment, the propellant is selected from the group consisting of a hydrocarbon, a chlorofluorocarbon, dimethyl ether, a hydrofluorocarbon, and mixtures thereof.

In one embodiment, the propellant is a mixture of hydrocarbons. In a further embodiment, the mixture of hydrocarbons is a mixture of propane, n-butane and isobutane.

The propellant is typically present in an amount from about 3% to about 15% by weight. In one embodiment, the propellant is present in an amount from about 5% to about 10% by weight, such as about 5%, 6%, 7%, 8%, 9% or 10% by weight.

The aerosol foam composition is packaged within a pressurized container, such as a standard aerosol dispenser.

When the composition is released from the pressurized container, the composition is an aerosol foam (also known as a mousse). In an embodiment, the aerosol foam breaks easily with shear, such as gentle mechanical action e.g. rubbing or spreading.

Standard aerosol dispensers for use herein include aluminium, tin-plate and glass containers.

In one embodiment, the pressurized container is a one-piece aluminium container in which the inner surface is lined with a chemically inert lining. One suitable inner surface lining for use herein is polyamide-imide (PAM), such as that supplied by Exal Corporation, of Youngstown, Ohio. The container may be fitted with an upright-use or inverted-use valve and a conventional foam spout actuator.

### Additional active agents

According to an embodiment of the present invention, the pharmaceutical compositions may comprise a second pharmaceutically active agent.

In an embodiment, the second pharmaceutically active agent is selected from the group consisting of an antibacterial agent, antifungal agent, corticosteroid and vitamin D analogue.

According to a further embodiment, the second pharmaceutically active agent is an antibacterial agent. Suitably, the antibacterial agent is selected from the group consisting of gentamicin, neomycin, streptomycin, cefpodoxime proxetil, clindamycin, lincomycin, erythromycin, bacitracin, gramicidin, vancomycin, doxycycline, minocycline, oxytetracycline, tetracycline, fosfomycin, fusidic acid, mupirocin, sulfacetamide, metronidazole, dapsone, triclosan, quaternary ammonium salts, silver sulfadiazine, and salts and esters thereof.

According to another embodiment, the second pharmaceutically active agent is an antifungal agent. Suitably, the antifungal agent is selected from the group consisting of echinocandins such as anidulafunin, caspofungin and micafungin; polyenes such as amphotericin B, candicidin, filipin, fungichromin, hachimycin, hamycin, lucensomycin, mepartricin, natamycin, nystatin, pecilocin, perimycin; allylamines such as butenafine, naftifine and terbinafine; imidazoles such as bifonazole, butoconazole, chlormidazole, cloconazole, clotrimazole, econazole, enilconazole, fenticonazole, flutrimazole, isoconazole, ketoconazole, lanoconazole, miconazole, neticonazole, omoconazole, oxiconazole nitrate, sertaconazole, sulconazole and tioconazole; thiocarbamates such as liranaftate, tolciclate, tolindate and tolnafate; triazoles such as albaconazole, pramiconazole, fluconazole, itraconazole, luliconazole, posaconazole, ravuconazole, saperconazole, terconazole and voriconazole; and other antifungal agents such as acrisorcin, amorolfine, biphenamine, bromosalicylchloranilide, buclosamide, calcium propionate, chlorphenesin, ciclopirox, cloxyquin, coparaffinate, exalamide, flucytosine, haloprogin, hexetidine, loflucarban, nifuratel, potassium iodide, propionic acid, pyrithione, salicylanilide, sodium propionate, sulbentine, tenonitrozole, triacetin, undecylenic acid, zinc propionate, griseofulvin, oligomycins, pyrrolnitrin, siccanin, viridian, and salts and esters thereof.

According to an embodiment, the antifungal agent is albaconazole.

According to one embodiment, the fatty acid monoester of glycerol is 1-decanoyl-rac-glycerol (i.e. 1-monocaprin) and the second pharmaceutically active agent is albaconazole.

According to another embodiment, the second pharmaceutically active agent is a corticosteroid. Suitably, the corticosteroid is selected from the group consisting of alclometasone, amcinonide, beclomethasone, betamethasone, budesonide, clobetasol, clobetasone, cortisone, desonide, desoximetasone, diflorasone, diflucortolone, fluclorolone, flumethasone, fluocinolone, fluocinonide, fluocortin butyl, fluocortolone, fluprednidene, flurandrenolide, flurandrenolone, fluticasone, halcinonide, halobetasol, hydrocortisone, methylprednisolone, mometasone, prednisone, triamcinolone acetonide, prednicarbate, and salts and esters thereof.

According to yet another embodiment, the second pharmaceutically active agent is a vitamin D analogue. Suitably, the vitamin D analogue is selected from the group consisting of calcidiol, calcitriol, calcipotriene, paricalcitol, 22-oxacolcitriol, dihydrotachysterol, calciferol, and salts and esters thereof.

The second pharmaceutically active agent is present in a therapeutically effective amount. According to an embodiment, the second pharmaceutically active agent is present in an amount from about 0.005% to about 15% by weight.

### Other ingredients

The present pharmaceutical compositions may comprise additional excipients, as is known in the art. Suitably, the excipient is selected from pH adjusting agents, humectants, film extenders, chelating agents, antioxidants, preservatives, plasticizers, penetration enhancers, fragrance, colorants, surfactants, emollients, gelling agents, radical scavengers, or a combination or mixture thereof.

### pH Adjusting agent

The present pharmaceutical compositions may further comprise a pH adjusting agent. In one embodiment, the pH adjusting agent is a base. Suitable pH adjusting bases include amines, bicarbonates, carbonates, and hydroxides such as alkali or alkaline earth metal hydroxides, as well as transition metal hydroxides. In another embodiment, the pH adjusting agent is an acid, an acid salt, or mixtures thereof. According to yet another embodiment, the pH adjusting agent is a buffer. Suitably, the buffer is selected from citrate/ citric acid, acetate/ acetic acid, phosphate/ phosphoric acid, formate/ formic acid, propionate/ propionic acid, lactate/ lactic acid, carbonate/ carbonic acid, ammonium/ ammonia, edetate/ edetic acid, or a combination or mixture thereof.

According to an embodiment, the pH adjusting agent is present in an amount from about 0.01% to about 10% by weight. According to another embodiment, the pH adjusting agent is present in an amount sufficient to adjust the pH of the composition to between about 4 to about 6.5.

### Humectants

The present pharmaceutical compositions may further comprise a humectant. Non-limiting examples of humectants useful in this regard include glycerol, sorbitol, maltitol, polydextrose, triacetin, propylene glycol, polyethylene glycol (PEG) esters including PEG-20 stearate, PEG-40 stearate, PEG-150 stearate, PEG-150 distearate and PEG-100 stearate, alkoxylated alcohols including laureth-12, ceteareth-20, laureth-23, glycereth-7, glycereth-12, glycereth-26, PEG-4, PEG-6, PEG-8, PEG-12, PEG-32, PEG-75, PEG-150, or a combination or mixture thereof. In a preferred embodiment, the humectant is glycerol.

In an embodiment, the present compositions comprise about 0.1 % to about 10% by weight of a humectant. In a further embodiment, the present compositions comprise about 0.5% to about 5% by weight of a humectant.

### Film extenders

The present pharmaceutical compositions, particularly the nail lacquer compositions, may further comprise at least one film extender. Non-limiting examples of film extenders useful in this regard include calcium carbonate, calcium phosphate, calcium stearate, magnesium stearate, zinc stearate, calcium sulfate, colloidal silicon dioxide, kaolin, magnesium carbonate, magnesium silicate, sodium stearyl fumarate, talc, titanium dioxide, zinc oxide, or a combination or mixture thereof. In an embodiment, the film extender is present in an amount from about 0.1% to about 2% by weight.

### Chelating agents

The present pharmaceutical compositions may further comprise a chelating agent. Non-limiting examples of chelating agents useful in this regard include citric acid, isopropyl (mono) citrate, stearyl citrate, lecithin citrate, gluconic acid, tartaric acid, oxalic acid, phosphoric acid, sodium tetrapyrophosphate, potassium monophosphate, sodium hexametaphosphate, calcium hexametaphosphate, sorbitol, glycine (aminoacetic acid), methyl glucamine, triethanolamine (trolamine), EDTA, DEG (dihydroxyethylglycine), DPTA (diethylene triamine pentaacetic acid), NTA (nitrilotriacetic acid), HEDTA (N-(hydroxyethyl)-ethylenetriaminetriacetic acid), aminocarboxylates, dimercaperol (BAL), larixinic acid (maltol), unidentate ligands (fluoride and cyanide ions), diphenylthiocarbazone, o-phenanthroline, barium diphenylamine sulfonate, sodium glucoheptonate, 8-hydroxyquinoline, olefin complexes (such as dicyclopentadienyl iron), porphyrins, phosponates, or a combination or mixture thereof. In an embodiment, the chelating agent is present in an amount from about 0.1 % to about 1% by weight.

### Antioxidants

The present pharmaceutical compositions may further comprise an antioxidant. Non-limiting examples of substances which can serve as the antioxidant herein include butylated hydroxytoluene, butylated hydroxyanisole, tocopherol, propyl gallate, vitamin E TPGS, or a combination or mixture thereof. In an embodiment, the present compositions comprise an antioxidant in an amount from about 0.001 % to about 1% by weight.

### Preservatives

The present pharmaceutical compositions may further comprise a preservative. Non-limiting examples of substances which can serve as the preservative herein include benzyl alcohol, diazolidinyl urea, methyl paraben, ethyl paraben, propyl paraben, butyl paraben, phenoxyethanol, sorbic acid, benzoic acid, salts thereof, or a combination or mixture thereof. In an embodiment, the present compositions comprise a preservative in an amount from about 0.01% to about 2% by weight.

### Methods of treatment

The present compositions are particularly effective in treating the specific dermatophyte *Trichophyton rubrum.* According to an embodiment, the present invention relates to pharmaceutical compositions comprising the compounds of formula Ia for use in the topical treatment or prophylaxis of a fungal disorder caused by a *Trichophyton rubrum* infection. In one embodiment, the fungal disorder is selected from the group consisting of onychomycosis, tinea pedis (athlete's foot), tinea cruris (groin) and dermatophytosis (ringworm). According to one embodiment, the fungal condition is onychomycosis. The present compositions are also effective in treating fungal conditions caused by *Trichophyton mentagrophytes* and *Epidermophyton floccosum.*

### Combination therapy

In another embodiment, the present compositions may be used in combination with an additional (separate) dosage form to enhance the treatment of the fungal condition. This additional dosage form may be applied or taken at the same time as the present compositions i.e. concomitantly. Alternatively, one of the present compositions and the additional dosage form is administered in the morning and the other is administered in the evening (or vice versa).

In an embodiment, the present composition is administered as a combination with a separate oral composition containing an antifungal agent, preferably an antifungal agent which is different to the fatty acid monoesters of the present invention. Exemplary antifungal agents suitable for use in the oral composition include, but are not limited to, terbinafine, albaconazole, pramiconazole, itraconazole, griseofulvin or fluconazole.

According to an alternative embodiment, the present pharmaceutical compositions are used as a maintenance therapy. Maintenance therapy is initiated following substantial or complete alleviation of the symptoms of the fungal condition following primary treatment. According to a particular embodiment, the composition for maintenance therapy is an aerosol foam comprising 1-decanoyl-rac-glycerol (C₁₀), i.e. 1-monocaprin.

### Definitions

As used herein, the terms "administered", "administering" and "administration," refer to any method which, in sound medical practice, delivers the composition to a patient in such a manner as to provide a therapeutic effect.

It should be understood that the terms "a" and "an" as used above and elsewhere herein refer to "one or more" of the enumerated components. It will be clear to one of ordinary skill in the art that the use of the singular includes the plural unless specifically stated otherwise. Therefore, the terms "a", "an" and "at least one" are used interchangeably in this application.

Throughout the application, descriptions of various embodiments use "comprising" language, however it will be understood by one of skill in the art, that in some specific instances, an embodiment can alternatively be described using the language "consisting essentially of" or "consisting of".

As used herein, the phrases an "effective amount" or a "therapeutically effective amount" of an active agent or ingredient, or pharmaceutically active agent or ingredient, which are synonymous herein, refer to an amount of the pharmaceutically active agent sufficient to have a therapeutic effect upon administration. A therapeutically effective amount of the pharmaceutically active agent may, will, or is expected to treat the fungal condition. Effective amounts of the pharmaceutically active agent will vary with the particular condition being treated, the severity of the condition, the duration of the treatment, and the specific components of the composition being used.

As used herein, the term "matrix" means the space within the crosslinked polymer structure. This space also serves as a "reservoir" wherein the active ingredient or ingredients reside prior to administration.

As used herein, the term "salts thereof" refers to salts that are pharmaceutically acceptable and that possess the desired pharmacological activity of the parent compound. Such salts include: (1) acid addition salts, formed with acids such as, for example, acetic acid, benzoic acid, citric acid, gluconic acid, glutamic acid, glutaric acid, glycolic acid, hydrochloric acid, lactic acid, maleic acid, malic acid, malonic acid, mandelic acid, phosphoric acid, propionic acid, sorbic acid, succinic acid, sulfuric acid, tartaric acid, naturally and synthetically derived amino acids, and mixtures thereof; or (2) salts formed when an acidic proton present in the parent compound is either (i) replaced by a metal ion e.g. an alkali metal ion, an alkaline earth metal ion, or an aluminium ion; or (ii) protonates an organic base such as, for example, ethanolamine, diethanolamine, triethanolamine, tromethamine and N-methylglucamine.

As used herein, the term "substantially free" of a specified component refers to a composition with less than about 1% of the specified component.

As used herein, a "treatment" or "treating" of a condition encompasses alleviation of at least one symptom thereof, a reduction in the severity thereof, or the delay, prevention or inhibition of the progression thereof. Treatment need not mean that the condition is totally cured. A useful composition herein need only to reduce the severity of a condition, reduce the severity of symptoms associated therewith, provide improvement to a patient's quality of life, or delay, prevent or inhibit the onset of a condition.

For the purposes of better understanding the present teachings and in no way limiting the scope thereof, unless otherwise indicated, all numbers expressing quantities, percentages or proportions, and other numerical values used in the specification and claims, are to be understood as being modified in all instances by the term "about".

Other terms as used herein are meant to be defined by their well-known meanings in the art. Unless defined otherwise, all technical and scientific terms used herein have the same meaning as commonly understood to one of ordinary skill in the art to which the presently described invention pertains.

The following Examples are illustrative of the present invention.

### BIOLOGICAL EXAMPLES

### Example 1- Liquid broth assay

The minimum inhibitory concentration (MIC) of various test agents were tested in a standard protocol against two dermatophytes: *Trichophyton rubrum* and *Trichophyton mentagrophytes:*
1. ciclopirox
2. terbinafine HCl
3. econazole Nitrate
4. tioconazole
5. ketoconazole
6. fusaric Acid
7. 1-monocaprin (1-decanoyl-rac-glycerol)

*Trichophyton rubrum* and *Trichophyton mentagrophytes* were grown on potato dextrose agar (PDA) at 30°C for 1-5 weeks. One clone was picked randomly and grown in sabouraud dextrose broth (SDB) to a density of 1 x 10⁶ colony forming units (cfu) per ml. Various concentrations of test agents were added to the dermatophyte cultures and incubated for up to 4 weeks. Each week, an aliquot of the liquid culture was taken and placed on a PDA plate, and incubated for up to 3 weeks to assess growth. The plates were examined and photographed each week. Cultures were considered positive when more than 2 colonies were found per plate.

The results of the anti-fungal activity assessment of various agents in liquid (broth) cultures of *Trichophyton rubrum* and *Trichophyton mentagrophytes* are presented in Figure 1.

The liquid broth assay results suggest an antifungal activity ranking for both dermatophytes as follows: 1) terbinafine and econazole, 2) tioconazole, 3) ketoconazole, 4) ciclopirox, 5) 1-monocaprin and 6) fusaric acid.

Terbinafine and econazole were the most active in this assay, and had minimal inhibitory concentrations (MICs) below the lowest concentration tested (< 3.9 µg/ml).

Fusaric acid was the least active with a MIC of 500 µg/ml.

1-monocaprin had a MIC of 250 µg/ml and ciclopirox had a MIC of 31.3 µg/ml.

### Example 2 - Infected nail model

The following example provides a comparison of the efficacy of the composition according to Table 1 a) with a commercial comparator, namely PENLAC® nail lacquer.

The assay used in the comparison was an *in vitro* infected nail model. The assay used *Trichophyton rubrum* infected cadaver nail samples to evaluate the efficacy of the test formulations. The investigations were conducted under conditions which are closer to the clinical situation and thus have more practical relevance than a liquid broth assay as described above. The assay uses levels of ATP recovered from viable organisms as a biological marker to demonstrate the effectiveness of different formulations in reducing the viability of fungal cells.

Figure 2 shows the variation in ATP release from *Trichophyton rubrum* infected nail samples, on application of the composition of Table 1a), the commercial comparator (PENLAC® nail lacquer), placebos and controls (all sets of experiments tested at n=6). It is observed that the ATP recovered from the infected nail samples on application of the composition of the present invention was significantly lower than the amount of ATP recovered from *Trichophyton rubrum* infected nail samples treated with PENLAC® nail lacquer. This shows that the compositions of the present invention are significantly more effective in reducing the viability of *Trichophyton rubrum* cells in the presence of *nail samples* compared to the commercial comparator.

### FORMULATION EXAMPLES

### Example 3 - 1-monocaprin nail lacquer

The following example illustrates nail lacquer compositions of the present invention, with w/w% provided:

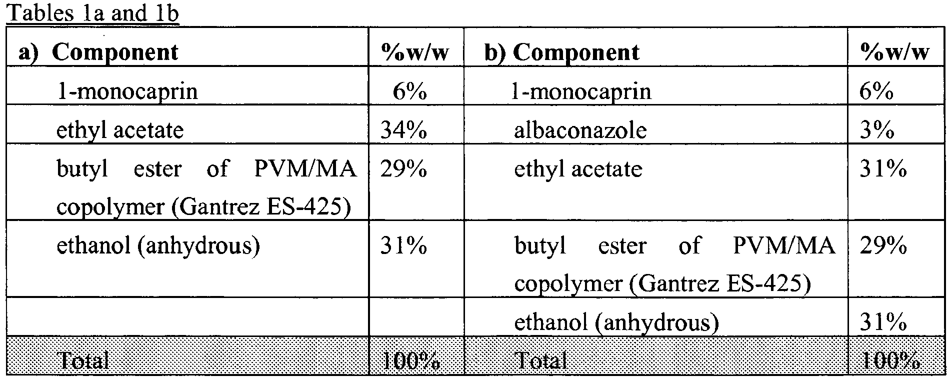

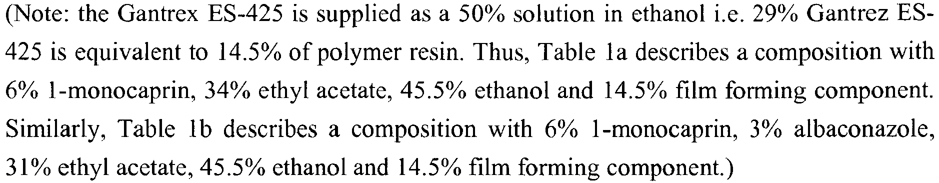

The nail lacquers were prepared as follows:
1. dissolve 1-monocaprin in the ethanol solvent whilst stirring (and then dissolve the second active ingredient in ethanol solvent, if applicable)
2. when the active ingredient(s) is fully dissolved, add ethyl acetate solvent, whilst stirring
3. add Gantrez ES-425 whilst stirring and stir until a clear solution results

### Example 4 - 1-monocaprin aerosol foam composition

The following example illustrates an aerosol emulsion foam composition according to the present invention. The foam is suitable for the primary treatment of onychomycosis, or as a maintenance therapy.

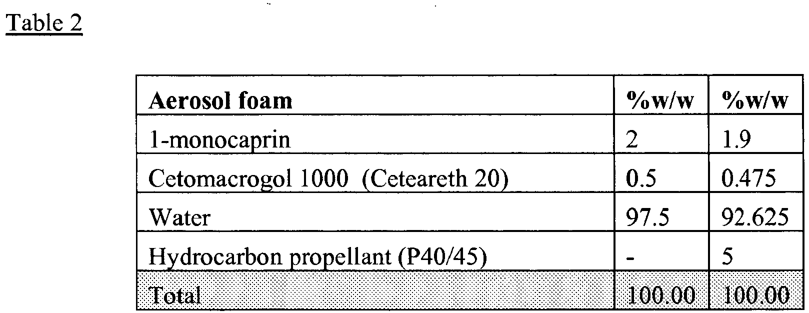

The aerosol foam base was prepared as follows:
1. warm 1-monocaprin (2%) with cetomacrogol 1000 (0.5%) until a clear solution results
2. add hot water (47.5 w/w) to the clear solution from step 1 to give a translucent, faintly opalescent solution
3. add cold water (50% w/w) to give a more opalescent solution which represents the foam base

### Preparation of aerosol foam:

transfer the foam base to an aerosol container, insert a valve, vacuum crimp the aerosol container and add a suitable hydrocarbon propellant to give an aerosol foam composition.

## Claims

1. A composition for use in the topical treatment or prophylaxis of a fungal condition caused by *Trichophyton rubrum* in a patient, the composition comprising a therapeutically effective amount of a fatty acid monoester of glycerol of the general formula Ia: 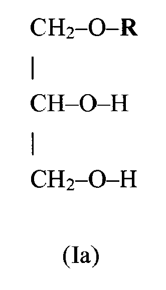 wherein **R** is a branched chain or straight chain acyl group having from 4 to 22 carbon atoms, and a pharmaceutically acceptable carrier or diluent thereof, and optionally a second pharmaceutically active agent.

2. The composition for use according to claim 1, wherein **R** is a straight chain acyl group having from 8 to 14 carbon atoms selected from the group consisting of -C(O)-C₇H₁₅, -C(O)-C₈H₁₇, -C(O)-C₉H₁₉, -C(O)-C₁₀H₂₁, -C(O)-C₁₁H₂₃, -C(O)-C₁₂H₂₅, and - C(O)-C₁₃H₂₇.

3. The composition for use according to claim 1 or 2, wherein **R** is a straight chain acyl group which is -C(O)-C₉H₁₉.

4. The composition for use according to any one of claims 1 to 3, wherein the fatty acid monoester of glycerol is present in an amount from 0.1 % to 20% by weight.

5. The composition for use according to any one of claims 1 to 4, wherein the composition is a nail lacquer.

6. The composition for use according to claim 5, wherein the composition is a nail lacquer and further comprises at least one volatile solvent and a film forming component.

7. The composition for use according to claim 6, wherein the at least one volatile solvent is a mixture of ethyl acetate and ethanol.

8. The composition for use according to claim 6 or 7, wherein the film forming component is the butyl ester of PVM/MA copolymer.

9. The composition for use according to any one of claims 1 to 4, wherein the composition is an aerosol foam.

10. The composition for use according to claim 9, wherein the composition is an aerosol foam and further comprises water, a surfactant component, and a propellant.

11. The composition for use according to any one of claims 1 to 10, wherein the composition comprises a second pharmaceutically active agent, preferably albaconazole.

12. The composition for use according to any one of claims 1 to 11, wherein the fungal condition is selected from the group consisting of tinea pedis, tinea cruis, dermatophysis and onychomycosis.

13. The composition for use according to claim 12, wherein the fungal condition is onychomycosis.

## Patentansprüche

1. Eine Zusammensetzung zur Verwendung bei der topischen Behandlung oder Prophylaxe eines pilzartigen Zustands, verursacht durch *Trichophyton rubrum,* bei einem Patienten, wobei die Zusammensetzung eine therapeutisch wirksame Menge eines Fettsäuremonoesters von Glycerin der allgemeinen Formel Ia: 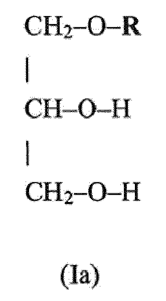 wobei **R** ein verzweigtkettiger oder geradkettiger Acylrest mit 4 bis 22 Kohlenstoffatomen ist, und einen pharmazeutisch verträglichen Träger oder ein pharmazeutisch verträgliches Verdünnungsmittel davon und gegebenenfalls einen zweiten pharmazeutischen Wirkstoff umfasst.

2. Die Zusammensetzung zur Verwendung nach Anspruch 1, wobei **R** ein geradkettiger Acylrest mit 8 bis 14 Kohlenstoffatomen, ausgewählt aus der Gruppe, bestehend aus -C(O)-C₇H₁₅, -C(O)-C₈H₁₇, -C(O)-C₉H₁₉, -C(O)-C₁₀H₂₁, -C(O)-C₁₁H₂₃, -C(O)-C₁₂H₂₅ und -C(O)-C₁₃H₂₇, ist.

3. Die Zusammensetzung zur Verwendung nach Anspruch 1 oder 2, wobei **R** ein geradkettiger Acylrest ist, welcher -C(O)-C₉H₁₉ ist.

4. Die Zusammensetzung zur Verwendung nach einem der Ansprüche 1 bis 3, wobei der Fettsäuremonoester von Glycerin in einer Menge von 0,1 Gew.-% bis 20 Gew.-% vorhanden ist.

5. Die Zusammensetzung zur Verwendung nach einem der Ansprüche 1 bis 4, wobei die Zusammensetzung ein Nagellack ist.

6. Die Zusammensetzung zur Verwendung nach Anspruch 5, wobei die Zusammensetzung ein Nagellack ist und ferner mindestens ein flüchtiges Lösungsmittel und einen filmbildenden Bestandteil umfasst.

7. Die Zusammensetzung zur Verwendung nach Anspruch 6, wobei das mindestens eine flüchtige Lösungsmittel ein Gemisch aus Ethylacetat und Ethanol ist.

8. Die Zusammensetzung zur Verwendung nach Anspruch 6 oder 7, wobei der filmbildende Bestandteil der Butylester von PVM/MA-Copolymer ist.

9. Die Zusammensetzung zur Verwendung nach einem der Ansprüche 1 bis 4, wobei die Zusammensetzung ein Aerosolschaum ist.

10. Die Zusammensetzung zur Verwendung nach Anspruch 9, wobei die Zusammensetzung ein Aerosolschaum ist und ferner Wasser, ein grenzflächenaktives Mittel als Bestandteil und ein Treibmittel umfasst.

11. Die Zusammensetzung zur Verwendung nach einem der Ansprüche 1 bis 10, wobei die Zusammensetzung einen zweiten pharmazeutischen Wirkstoff, bevorzugt Albaconazol, umfasst.

12. Die Zusammensetzung zur Verwendung nach einem der Ansprüche 1 bis 11, wobei der pilzartige Zustand aus der Gruppe, bestehend aus Tinea pedis, Tinea cruris, Dermatophytose und Onychomykose, ausgewählt ist.

13. Die Zusammensetzung zur Verwendung nach Anspruch 12, wobei der pilzartige Zustand Onychomykose ist.

## Revendications

1. Composition pour une utilisation dans le traitement topique ou la prophylaxie d'une affection fongique provoquée par *Tricophyton rubrum* chez un patient, la composition comprenant une quantité thérapeutiquement efficace d'un mono-ester d'acide gras de glycérol de formule générale Ia : 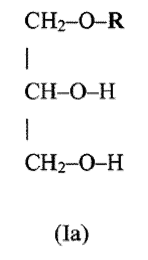 dans laquelle **R** représente un groupe acyle à chaîne ramifiée ou à chaîne droite ayant 4 à 22 atomes de carbone, et un support ou diluant pharmaceutiquement acceptable à cette fin, et éventuellement un second agent pharmaceutiquement actif.

2. Composition pour une utilisation suivant la revendication 1, dans laquelle **R** représente un groupe acyle à chaîne droite ayant 8 à 14 atomes de carbone, choisi dans le groupe consistant en des groupes -C(O)-C₇H₁₅, -C(O)C₈H₁₇, -C(O)C₉H₁₉. -C(O)C₁₀H₂₁, -C(O)C₁₁H₂₃, -C(O)C₁₂H₂₅ et -C(O)C₁₃H₂₇.

3. Composition pour une utilisation suivant la revendication 1 ou 2, dans laquelle R représente un groupe acyle à chaîne droite qui est un groupe -C(O)C₉H₁₉.

4. Composition pour une utilisation suivant l'une quelconque des revendications 1 à 3, dans laquelle le mono-ester d'acide gras de glycérol est présent en une quantité de 0,1 % à 10 % en poids.

5. Composition pour une utilisation suivant l'une quelconque des revendications 1 à 4, ladite composition étant un vernis à ongles.

6. Composition pour une utilisation suivant la revendication 5, la composition étant un vernis à ongles et comprenant en outre au moins un solvant volatil et un constituant filmogène.

7. Composition pour une utilisation suivant la revendication 6, dans laquelle ledit au moins un solvant volatil est un mélange d'acétate d'éthyle et d'éthanol.

8. Composition pour une utilisation suivant la revendication 6 ou 7, dans laquelle le constituant filmogène est l'ester butylique de copolymère PVM/MA.

9. Composition pour une utilisation suivant l'une quelconque des revendications 1 à 4, ladite composition étant une mousse en aérosol.

10. Composition pour une utilisation suivant la revendication 9, la composition étant une mousse en aérosol et comprenant en outre de l'eau, un constituant tensioactif et un agent propulseur.

11. Composition pour une utilisation suivant l'une quelconque des revendications 1 à 10, ladite composition comprenant un second agent pharmaceutiquement actif, de préférence l'albaconazole.

12. Composition pour une utilisation suivant l'une quelconque des revendications 1 à 11, l'affection fongique étant choisie dans le groupe consistant en la teigne des pieds, l'eczéma marginé, la dermatophytie et l'onychomycose.

13. Composition pour une utilisation suivant la revendication 12, l'affection fongique étant l'onychomycose.
